# EUROPEAN PATENT APPLICATION

(11) **EP 1 544 195 A1**
(43) Date of publication of application: **22.06.2005**
(21) Application number: 03293152.9
(22) Date of filing: 15.12.2003
(51) Int. Cl.: C07D 233/56, C07D 419/12, A61K 31/4164, A61K 31/5415, A61P 5/00, A61P 35/00

(54) **1-N-phenyl-amino- 1H-imidazole derivatives and pharmaceutical compositions containing them**

(71) Applicant: LABORATOIRE THERAMEX, 98000 Monaco (MC)
(72) Inventor: Lafay, Jean, 06100 Nice (FR); Rondot, Benoît, 06480 La Colle sur Loup (FR); Bonnet, Paule, 06500 Menton (FR); Clerc, Thierry, Les Balcons d'Eze, 06360 Eze (FR); Shields, Jacqueline, 06100 Nice (FR); Duc, Igor, 06400 Cannes (FR); Duranti, Eric, 06700 Saint Laurent du Var (FR); Puccio, François, 06300 Nice (FR); Blot, Christian, Rés. "Les Closeries", 06480 La Colle sur Loup (FR); Maillos, Philippe, 06300 Nice (FR)
(74) Representative: Gillard, Marie-Louise

(57) **Abstract**

The invention relates to the compounds of formula (I): 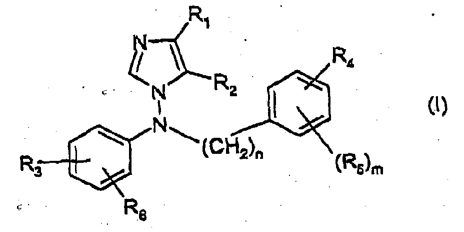 In which R₁, R₂, R₃, R₄, R₅, R₆, m and n are as defined in the specification, which are potent aromatase and/or steroid sulfatase and/or carbonic anhydrase inhibitors and generally relate to the field of hormone- and non hormone- dependent cancer and endocrine disorders.
The invention also relates to the pharmaceutical compositions containing these compounds.

## Description

The present invention relates to 1-N-phenyl-amino-1H-imidazole derivatives and to pharmaceutical compositions containing them.

The invention generally relates to the field of hormone- and non hormone-dependent cancer and endocrine disorders.

Aromatase is the physiological enzyme responsible for the specific conversion of androgens such as androstenedione or testosterone, into estrogens such as estrone and estradiol, respectively (Simpson ER et al., Endocrine Reviews, 1994, 15 : 342-355). Inhibition of aromatase is, therefore, a strategy of choice to interfere with normal or pathological estrogen-induced or estrogen-dependent biological processes such as female sexual differentiation, ovulation, implantation, pregnancy, breast and endometrial cell proliferation as well as regulations of spermatogenesis or prostate cell proliferation in male or of non-reproductive functions such as bone formation or immune T cell and cytokine balance (see Simpson ER et al., Recent Progress in Hormone Research, 1997, 52 : 185-213 and the whole issues of Endocrine Related Cancer (1999, volume 6, n°2) and Breast Cancer Research Treatment (1998, volume 49, supplement n°1)).

The enzyme steroid sulfatase (E.C. 3.1.6.2., STS) catalyses the hydrolysis of estrone sulfate to estrone and the DHEA sulfate to DHEA (Dibbelt L, Biol. Chem, Hoppe-Seyler, 1991, 372, 173-185 and Stein C, J. Biol. Chem., 1989, 264, 13865-13872).

The steroid sulfatase pathway has been the focus of recent interest in the context of breast cancer, with regard to the local intra-tissue formation of estrogens from the abundant circulating pool of estrone sulfate (E₁S) (Pasqualini JR, J. Steroid Biochem. Mol. Biol., 1999, 69, 287-292 and Purohit A, Mol. Cell. Endocrine)., 2001, 171, 129-135).

Inhibition of this enzyme would prevent E₁S from yielding free estrone (E₁), which can be transformed into estradiol (E₂) by enzymatic reduction. In addition to the estrone sulfatase pathway, it is now believed that another potent estrogen, androstenediol (adiol) obtained from DHEA after hydrolysis of DHEA-S, could be another important contributor, in the support of growth and development of hormone-dependent breast tumors.

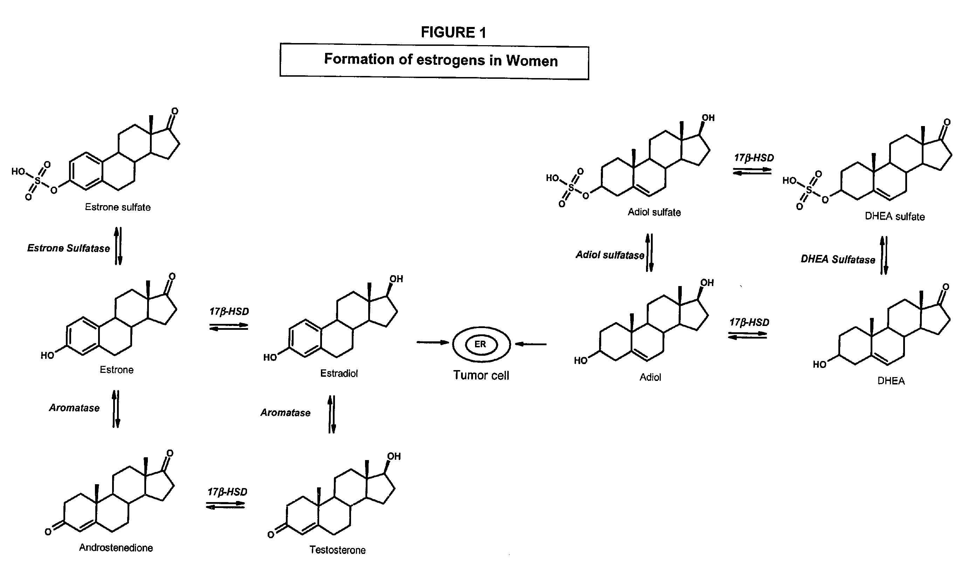

The formation of estrogens in women is schematically represented in figure 1.

In patients with hormone-dependent cancers, aromatase inhibitors are currently used to prevent estrogen synthesis. However, clinical trials showed a relative lack of efficacy for patients with estrogen receptor-positive tumors (Castiglione-Gertsch M, Eur. J. Cancer, 1996, 32A, 393-395 and Jonat W, Eur. J. Cancer, 1996, 32A, 404-412). As an explanation, steroid sulfatase pathway could be another important route for estrogen formation in breast tumors.

EMATE (Ahmed S, Curr. Med. Chem., 2002, 9, 2, 263-273), estrone-3-sulfamate, is the historical standard steroidal sulfatase inhibitor but has the major drawback of being estrogenic because of its mechanism of inhibition: the sulfamate moiety is cleaved during the process of enzyme inactivation, which releases E₁, not from E₁S but from EMATE itself (Ahmed S, J. Steroid Biochem. Mol. Biol., 2002, 80, 429-440).

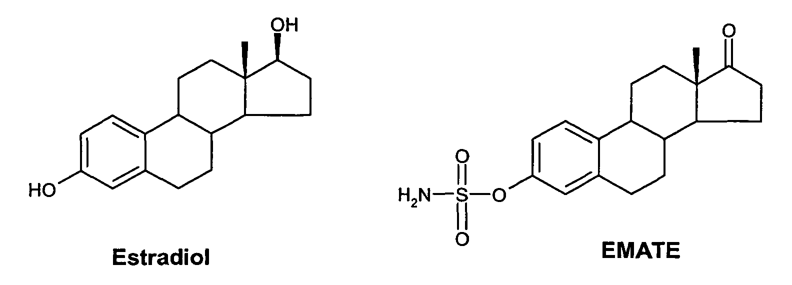

Other non-steroid sulfamate compounds which release derivatives without estrogenic properties have been presented as acceptable drug candidates such as 6,6,7-COUMATE, a standard non-estrogenic sulfatase inhibitor from the literature (Purohit A, Cancer Res, 2000, 60, 3394-3396).

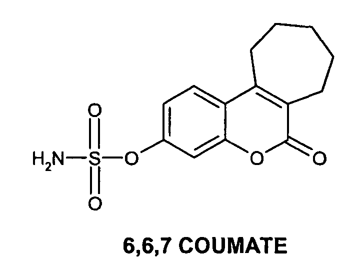

Human carbonic anhydrases catalyse the conversion between carbon dioxide (CO₂) and the bicarbonate ion (HCO₃⁻), and are involved in physiological and pathological processes. They include hormone-dependent and non-hormone-dependent cancerogenesis, metastasis invasive process and hypoxic tumors that express these enzymes which are less responsive to classical chemo/radio-therapy inhibitors. In particular, EMATE was found to have a human carbonic anhydrase inhibitory potency similar to that of acetazolamide, a well-known sulfonamide human carbonic anhydrase inhibitor (Winum J and al., J. Med. Chem. 2003, 46, 2197-2204).

It is therefore of particular interest to have compounds with at least one, preferably at least two of the following activities: aromatase inhibition, steroid sulfatase inhibition and carbonic anhydrase inhibition.

M. Okada et al. (Chem. Pharm. Bull., 44 (10), 1996, 1871-1879) described a series of [4-(bromophenylmethyl)-4-(cyanophenyl)-amino]-azoles and their azine analogues presented as aromatase inhibitors:

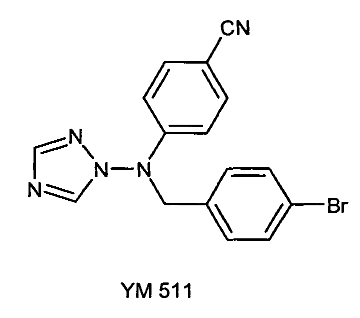

More recently, B. Potter et al. (J. Med. Chem., 46, 2003, 3193-3196) reported that sulfamoylated-derivatives of the aromatase inhibitor YM 511 inhibited sulfatase and aromatase activity in JEG-3 cells.

It has now been found that imidazole derivatives which invariably contain a 1-[N-phenylamino] group demonstrate an unexpectedly high potency to inhibit aromatase and/or steroid sulfatase and/or carbonic anhydrase.

Accordingly, one object of this invention is to provide 1-[N-phenyl-amino]-imidazole derivatives, which are potent aromatase and/or steroid sulfatase and/or carbonic anhydrase inhibitors.

Another object of this invention is to provide a pharmaceutical composition containing, as active ingredient, a 1-[N-phenylamino]-imidazole derivative as depicted below.

A further object of this invention is to provide the use of 1-[N-phenylamino]-imidazole derivatives in the manufacture of a medicament for treating or preventing various diseases and for managing reproductive functions in women, in men as well as in female and male wild or domestic animals.

The 1-[N-phenylamino]-imidazole derivatives of this invention are represented by the following general formula (I): 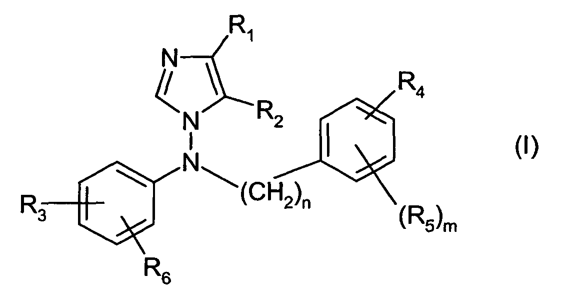 and acid addition salts and stereoisomeric forms thereof, wherein:
- R₁ and R₂ are each independently hydrogen, a (C₁-C₆)alkyl or a (C₃-C₈)cycloalkyl ; or R₁ and R₂ together form a saturated or unsaturated 5-, 6- or 7- membered carbocyclic ring;
- n is 0, 1 or 2 ;
- one of R₃ and R₄ is a hydroxy, nitro, -NR₇R₈, -OSO₂NR₇R₈ or -NR₇SO₂NR₇R₈ group and the other is hydrogen or a hydroxy, cyano, halogen, nitro, (C₁-C₆)alkyl, (C₁-C₆)alkoxy, trifluoromethyl, (C₁-C₆)alkylthio, (C₁-C₆)alkylsulfonyl, acyl, (C₁-C₆)alkoxycarbonyl, carboxamido, -NR₇R₈, -OSO₂NR₇R₈ or - NR₇SO₂NR₇R₈ group;
- R₅ and R₆ are each independently hydroxy, cyano, halogen, nitro, (C₁- ₆)alkyl, (C₁-C₆)alkoxy, trifluoromethyl, (C₁-C₆)alkylthio, (C₁-C₆)alkylsulfonyl, acyl, (C₁-C₆)alkoxycarbonyl, carboxamido, -NR₇R₈, -OSO₂NR₇R₈ or - NR₇SO₂NR₇R₈ group ;
- R₇ and R₈ are each independently hydrogen, a (C₁-C₆)alkyl or a (C₃-C₈)cycloalkyl;
- m is 1, 2, 3 or 4;
- R₄ and R₅ together with the phenyl ring bearing them can also form a benzoxathiazine dioxide or a dihydrobenzoxathiazine dioxide;
- R₃ and R₆ together with the phenyl ring bearing them can also form a benzofurane or a N-methylbenzotriazole.

In the description and claims, the term "(C₁-C₆)alkyl" is understood as meaning a linear or branched hydrocarbon chain having 1 to 6 carbon atoms. A (C₁-C₆)alkyl radical is for example a methyl, ethyl, propyl, isopropyl, butyl, isobutyl, tert-butyl, pentyl, isopentyl or hexyl radical. Preferred alkyl radicals are those having 1, 2 or 3 carbon atoms.

The term "halogen" is understood as meaning a chlorine, bromine, iodine or fluorine atom.

The term "(C₃-C₈)cycloalkyl" is understood as meaning a saturated monocyclic hydrocarbon having 3 to 8 carbon atoms. A (C₃-C₈)cycloalkyl radical is for example a cyclopropyl, cyclobutyl, cyclopentyl, cyclohexyl, cycloheptyl or cyclooctyl radical.

The term "(C₁-C₆)alkoxy" is understood as meaning a group OR in which R is a (C₁-C₆)alkyl as defined above. A (C₁-C₆)alkoxy radical is for example a methoxy, ethoxy, propoxy, isopropoxy, butoxy, isobutoxy, tert-butoxy, n-pentyloxy or isopentyloxy radical. Preferred alkoxy radicals are those having 1, 2 or 3 carbon atoms.

The term "acyl" is understood as meaning a group 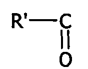 in which R' is hydrogen or a (C₁-C₄)alkyl wherein the term "alkyl" is as defined above. An acyl radical is for example a formyl, an acetyl, a propionyl, a butyryl or a valeryl radical. Preferred acyl radicals are formyl and acetyl.

Needless to say, when R₄ and R₅ together with the phenyl ring bearing them form a (dihydro)benzoxathiazine dioxide, then R₃ represents a hydroxy, nitro, -NR₇R₈, -OSO₂NR₇R₈ or -NR₇SO₂NR₇R₈ group. Likewise, when R₃ and R₆ together with the phenyl ring bearing them form a benzofurane or a N-methylbenzotriazole, then R₄ represents a hydroxy, nitro, -NR₇R₈, -OSO₂NR₇R₈ or -NR₇SO₂NR₇R₈ group. When R₃ and/or R₄ represent a group -NR₇SO₂NR₇R₈, the R₇ may be the same or different.

Compounds of formula (I) form acid addition salts, for example with inorganic acids such as hydrochloric acid, hydrobromic acid, sulfuric acid, nitric acid, phosphoric acid and the like or with organic carboxylic acids such as acetic acid, propionic acid, glycolic acid, pyruvic acid, oxalic acid, malic acid, fumaric acid, tartaric acid, citric acid, benzoic acid, cinnamic acid, mandelic acid, methanesulfonic acid and the like. Especially preferred are those salts which are pharmaceutically acceptable.

Preferred compounds of formula (I) comprise those fulfilling at least one of the following conditions:
- n is 0 or 1;
- R₁ and R₂ are each independently hydrogen or (C₁-C₆)alkyl;
- R₅ and R₆ are each independently hydrogen, halogen, (C₁-C₆)alkoxy, acyl, - NR₇R₈, -OSO₂NR₇R₈ or -NR₇SO₂NR₇R₈.

Further preferred compounds of formula (I) are those where:
- n is 0 or 1 ;
- R₁, R₂ and R₆ are each hydrogen;
- R₅ is hydrogen, halogen, (C₁-C₆)alkoxy or -OSO₂NR₇R₈.

Among the above compounds, those where one of R₃ and R₄ is hydroxy, cyano or -OSO₂NR₇R₈ and the other is hydroxy, nitro, -NR₇R₈, -OSO₂NR₇R₈ or -NR₇SO₂NR₇R₈, and preferably one of R₃ and R₄ is cyano or -OSO₂NR₇R₈ and the other is hydroxy or -OSO₂NR₇R₈, are especially preferred.

Also preferred are the compounds of formula (I) wherein: R₇ and R₈ are each hydrogen; n is 1; m is 1; R₃ is in position 4- of the phenyl ring; and/or R₄ is in position 4- of the phenyl ring.

By virtue of their capability to inhibit the enzymes aromatase and/or steroid sulfatase and/or carbonic anhydrase, the compounds of the present invention can be used alone or in combination with other active ingredients for the treatment or the prevention of any hormone or non hormone-dependent cancer, in humans as well as in wild or domestic animals. Because of their inhibition activity of aromatase and/or steroid sulfatase, the compounds of formula (I) are suitable for the management of estrogen-regulated reproductive functions, in humans as well as in wild or domestic animals.

In the treatment or prevention of the above conditions, the compounds of formula (I) can be used alone or in combination with an antiestrogen, a SERM (selective estrogen receptor modulator), an aromatase inhibitor, a carbonic anhydrase inhibitor, an antiandrogen, a steroid sulfatase inhibitor, a lyase inhibitor, a progestin, or a LH-RH agonist or antagonist. The compounds of formula (I) can also be used in combination with a radiotherapeutic agent; a chemiotherapeutic agent such as a nitrogenated mustard analogue like cyclophosphamide, melphalan, iphosphamide, or trophosphamide; an ethylenimine like thiotepa; a nitrosourea like carmustine; a lysed agent like temozolomide or dacarbazine; an antimetabolite of folic acid like methotrexate or raltitrexed; a purine analogue like thioguanine, cladribine or fludarabine; a pyrimidine analogue like fluorouracil, tegafur or gemcitabine; an alkaloid of vinca or analogue like vinblastine, vincristine or vinorelbine; a podophyllotoxin derivative like etoposide, taxanes, docetaxel or paclitaxel; an anthracycline or analogue like doxorubicin, epirubicin, idarubicin or mitoxantrone; a cytotoxic antibiotic like bleomycin or mitomycin; a platinum compound like cisplatin, carboplatin or oxaliplatin; a monoclonal antibody like rituximab; an antineoplastic agent like pentostatin, miltefosine, estramustine, topotecan, irinotecan or bicalutamide; or with a prostaglandin inhibitor (COX 2/COX 1 inhibitor).

The compounds of the invention can also be used for the control or management of estrogen-regulated reproductive functions such as male or female fertility, pregnancy, abortion or delivery, in humans as well as in wild or domestic animal species, alone or in combination with one or several other therapeutic agents such as a LH-RH agonist or antagonist, an estroprogestative contraceptive, a progestin, an antiprogestin or a prostaglandin inhibitor.

Breast tissue being a sensitive target of estrogen-stimulated proliferation and/or differentiation, inhibitors of aromatase and/or steroid sulfatase and/or carbonic anhydrase can be used in the treatment or prevention of benign breast diseases in women, gynecomastia in men and in benign or malignant breast tumors with or without metastasis both in men and women or in male or female domestic animals. The compounds of the invention can also be used in the treatment or prevention of benign or malignant disease of the uterus or the ovary. In each case, the compounds of the invention can be used alone or in combination with one or several other sexual endocrine therapeutic agents such as an antiandrogen, an anti-estrogen, a progestin or a LH-RH agonist or antagonist.

As the enzyme steroid sulfatase transforms DHEA sulfate into DHEA, a precursor of active androgens (testosterone and dihydrotestosterone), the compounds of the invention can be used in the treatment or prevention of androgen-dependent diseases such as androgenic alopecia (male pattern loss) (Hoffman R et al., J. Invest. Dermatol., 2001, 117, 1342-1348), hirsutism, acne (Billich A et al., WO 9952890), benign or malignant diseases of the prostate or the testis (Reed MJ, Rev. Endocr. Relat. Cancer, 1993, 45, 51-62), alone or in combination with one or several other sexual endocrine therapeutic agents such as an antiandrogen, an antiestrogen, a SERM, an antiaromatase, a progestin, a lyase inhibitor or a LH-RH agonist or antagonist.

Inhibitors of steroid sulfatase are also potentially involved in the treatment of cognitive dysfunction, because they are able to enhance learning and spatial memory in the rat (Johnson DA, Brain Res, 2000, 865, 286-290). DHEA sulfate as a neurosteroid affects a number of neurotransmitter systems including those involving acetylcholine, glutamate, and GABA, resulting in increased neuronal excitability (Wolf OT, Brain Res. Rev, 1999, 30, 264-288). The compounds of the present invention are thus also useful for enhancing the cognitive function, especially for the treatment of senile dementia, including Alzheimer's diseases, by increasing the DHEA levels in the central nervous system.

In addition, estrogens are involved in the regulation of the balance between Th₁ and Th₂ predominant immune functions and may therefore be useful in the treatment or prevention of gender-dependent auto-immune diseases such as lupus, multiple sclerosis, rheumatoid arthritis and the like (Daynes RA, J. Exp. Med, 1990, 171, 979-996). Steroid sulfatase inhibition was further shown to be protective in models of contact allergy and collagen-induced arthritis in rodents (Suitters AJ, Immunology, 1997, 91, 314-321).

Studies using 2-MeOEMATE have shown that steroid sulfatase inhibitors have potent estradiol-independent growth-inhibitory effect (MacCarthy-Moorogh L, Cancer Research, 2000, 60, 5441-5450). A decrease in tumor volume was surprisingly observed with the compounds of the invention, with low tumor steroid sulfatase inhibition. In view of this, the compounds of the invention could lead to a decrease in cellular division because of the large interaction between such new chemical entities and the microtubular network within the cancerous cell, whatever the tissue, including breast, endometrium, uterus, prostate, testis or metastasis generated therefrom. The compounds of the invention could therefore be useful in the treatment of non-estrogeno-dependent cancer.

The compounds of the invention are of particular value for the treatment or prevention of estrogen-dependent diseases or disorders, i.e. estrogen-induced or estrogen-stimulated diseases or disorders (Golob T, Bioorg. Med. Chem., 2002, 10, 3941-3953).

In addition, the compounds of the present invention are inhibitors of carbonic anhydrase (CA). This property could explain the interest of such compounds in non-hormone-dependent cancer. Immunohistochemical studies of CA II have shown that it is expressed in malignant brain tumors (Parkkila A-K. et al., Histochem. J., 1995, 27: 974-982) and gastric and pancreatic carcinomas (Parkkila S et al., Histochem. J., 1995, 27: 133-138), and recent evidence has shown that CA IX and XII are also expressed in some tumors and may be functionally related to oncogenesis. Ivanov et al. (Proc. Natl. Acad. Sci. USA, 1998, 95: 12596-12601) recently hypothesized that tumor-associated CA IX and

XII may be implicated in acidification of the extracellular medium surrounding cancer cells, which would create a microenvironment conducive to tumor growth and spreading. It has been shown that acetazolamide markedly inhibited invasion capacity in four renal cancer cell lines (Parkkila S et al., Proc. Natl. Acad. Sci. USA, 2000, 97: 2220-2224), an effect attributable to CA II, IX, and XII, which were expressed in these cells. Leukemia cells can easily spread from bone marrow to other organs via circulation, but various leukemias differ in their ability to form extramedullary tumors i.e., metastases. If CA activities were essential for invasion by other cancer cells, one could analogously predict that active CA(s) could also function in leukemia cells.

As used herein, the term "combined" or "combination" refers to any protocol for the co-administration of a compound of formula (I) and one or more other pharmaceutical substances, irrespective of the nature of the time of administration and the variation of dose over time of any of the substances. The co-administration can for example be parallel or sequential.

For the treatment/prevention of any of the above-mentioned diseases or disorders, the compounds of formula (I) may be administered, for example, orally, topically, parenterally, in dosage unit formulations containing conventional non-toxic pharmaceutically acceptable carriers, adjuvants and vehicles. These dosage forms are given as examples, but other dosage forms may be developed by those skilled in the art of formulation, for the administration of the compounds of formula (I). The term parenteral as used herein includes subcutaneous injections, intravenous, intramuscular, intrasternal injection or infusion techniques. In addition to the treatment of humans, the compounds of the invention are effective in the treatment of warm-blooded animals such as mice, rats, horses, cattle sheep, dogs, cats, etc.

The pharmaceutical compositions containing the active ingredient(s) may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient(s) in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

They may also be coated by the technique described in the U.S. Patent 4,256,108; 4,166,452 and 4,265,874 to form osmotic therapeutic tablets for controlled release.

Formulations for oral use may also be presented as hard gelatin capsules wherein the active ingredient(s) is (are) mixed with an inert solid diluent, for example, calcium carbonate, calcium phosphate, or kaolin, or as soft gelatin capsules wherein the active ingredient is mixed with water or an oil medium, for example peanut oil, liquid paraffin, or olive oil.

Aqueous suspensions contain the active ingredient(s) in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose, saccharin or aspartame.

Oily suspensions may be formulated by suspending the active ingredient(s) in a vegetable oil, for example peanut oil, olive oil, sesame oil or coconut oil, or in mineral oil such as liquid paraffin. The oily suspensions may contain a thickening agent, for example beeswax, hard paraffin or acetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient(s) in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present. The pharmaceutical compositions of the invention may also be in the form of an oil-in-water emulsion. The oily phase may be a vegetable oil, for example olive oil or peanut oil, or a mineral oil, for example liquid paraffin or mixtures of these. Suitable emulsifying agents may be naturally occurring phosphatides, for example soybean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavouring agents.

The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Dosage levels of the order of from about 0.0001 mg to about 20 mg/kg of body weight per day are useful in the treatment of the above-indicated conditions, or alternatively about 0.1 mg to about 2000 mg per patient per day.

The amount of active ingredient that may be combined with the carrier materials to produce a single dosage form will vary depending upon the host treated and the particular mode of administration. Dosage unit forms will generally contain between from about 0.1 mg to about 400 mg of compound of formula (I), typically 0.1 mg, 1 mg, 2 mg, 5 mg, 10 mg, 20 mg, 40 mg, 80 mg, 100 mg, 200 mg or 400 mg.

It will be understood, however, that the specific dose level for any particular patient will depend upon a variety of factors including the age, body weight, general health, sex, diet, time of administration, route of administration, rate of excretion, drug combination and the severity of the particular disease undergoing therapy.

According to another object, the invention relates to a method for the treatment or prevention of the above-mentioned diseases, disorders or conditions. The method comprises administering to a subject (human or animal) in need thereof a therapeutically effective amount of a compound of formula (I) or a pharmaceutically acceptable acid addition salt thereof.

The 1-N-phenyl-amino-1H-imidazole derivatives of formula (I) and their acid addition salts can be prepared following the general scheme 1.

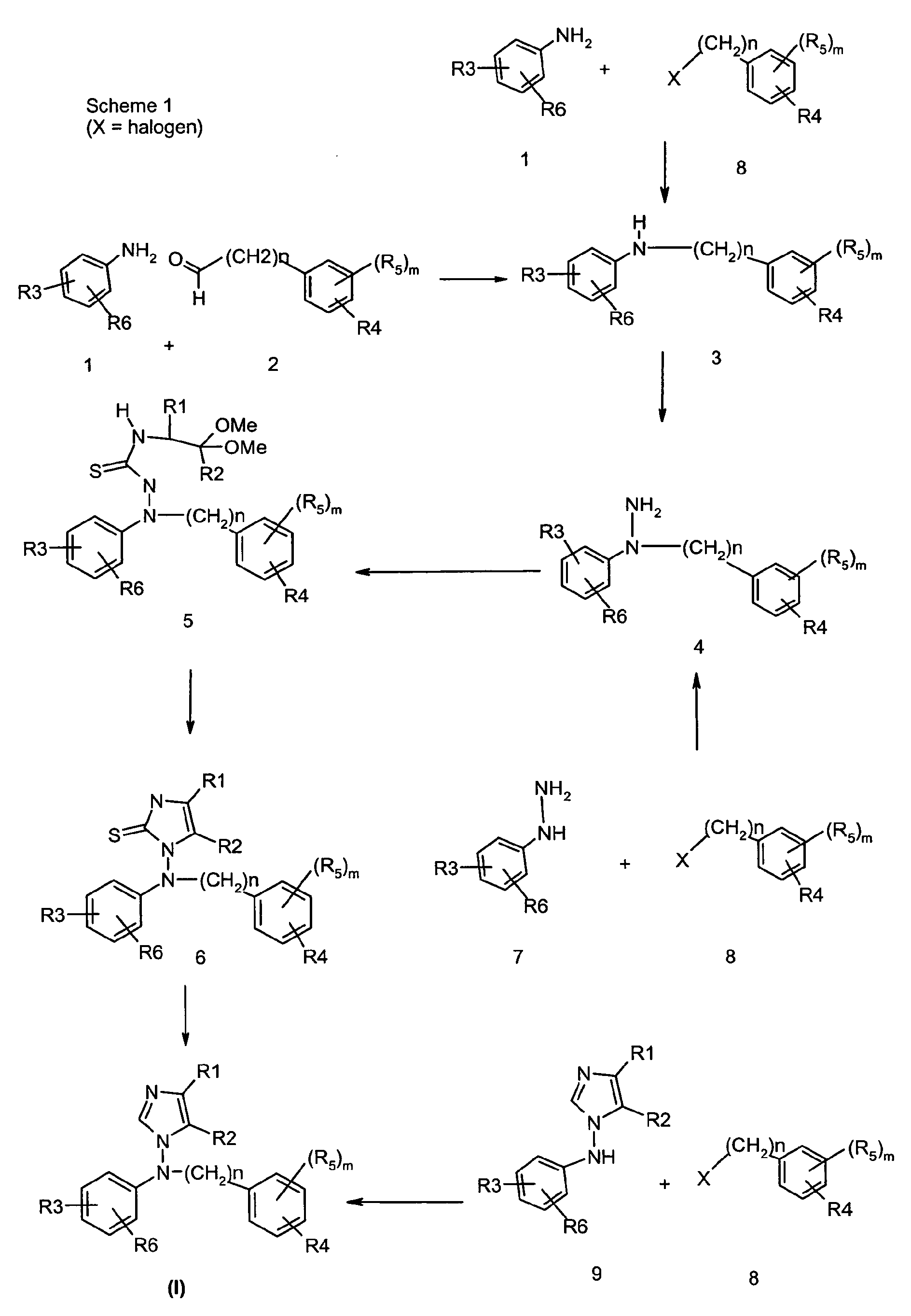

According to scheme 1 aniline derivative (1) is condensed with the aldehyde of formula (2) and the imine intermediate is reduced with sodium borohydride or hydrogenated using palladium or platinum oxide as catalyst to afford the N,N-disubstituted aniline (3). Said aniline (3) can also be prepared by reaction of a halogeno derivative (8) with an aniline of formula (1).

The N,N-disubstituted aniline (3) is converted to its nitroso derivative using standard conditions, then reduced to afford the 1,1-disubstituted hydrazine of formula (4).

Alternatively, the 1,1-disubstituted hydrazine (4) can be prepared by selective N-alkylation of a compound of formula (7) with a compound of formula (8) using the conditions described by U. Lerch and J. König (*Synthesis,* 1983, 2, 157-8) or the conditions described by J. Chung et al. (*Tetrahedron Letters,* 1992, 33, 4717-20).

Then, condensation of (4) with a dialkyloxy-alkyl-isothiocyanate derivative or an ethylenedioxy-alkyl-isocyanate derivative, affords the thiosemicarbazide (5) which is transformed to the 1-amino-imidazole-2-thione (6) by treatment with an acid like acetic acid or sulphuric acid.

Desulfurization of (6) in acetic acid, following the conditions described by S.Grivas and E.Ronne in *Acta Chemica Scandinavia,* 1995, 49, 225-229, gives the final 1-N-phenyl-amino-1H-imidazole (I), which is optionally converted to one of its acid addition salts. Alternatively the compounds (I) where R₃ or R₆ is an electron-withdrawing group can be obtained by condensation of the N-imidazoloaniline (9) with the halogeno derivative (8).

Deprotection of the derivatives (I) where R₃ or R₄ is alkoxy e.g. methoxy, with boron tribromide gives the corresponding hydroxy compounds prepared using the conditions described by McOmie J.F.W. (Tetrahedron, 1968, 24, 2289-92).

These compounds are transformed into the corresponding sulfamates by treatment with sodium hydride, with amidochlorosulfonic acid (Nussbaumer P, J.

Med. Chem., 2002, 45, 4310-20), or by reaction with sulfamoyl chloride in dimethylacetamide (DMAc) (Makoto. O, Tetrahedron letters, 2000, 41, 7047-51).

The derivatives (I) where R₃ and/or R₄ is nitro are reduced with ruthenium and hydrazine, to give the corresponding amino compounds (WO 02051821). These compounds are transformed into the corresponding sulfamides by the same treatment as described for the conversion into sulfamates.

The following examples are intended to illustrate and not to limit the scope of the invention.

### PREPARATION OF N, N-DISUBSTITUTED HYDRAZINES (4)

### EXAMPLE 1

### N¹-(4-cyanophenylmethyl)-N¹-(4-methoxyphenyl)hydrazine

Chloromethylbenzonitrile (25 g, 164.90 mmol) was introduced with stirring into a flask containing toluene (200 ml) and triethylamine (46.40 ml, 329.80 mmol). 4-cyano-phenylhydrazine hydrochloride (prepared following José L. Castro et al. J.Med.Chem. 1994, 37, 3023-3032) (28.80 g, 164.90 mmol) was added portionwise and the reaction mixture was stirred 3 h at reflux. After cooling, the mixture was filtered, washed with toluene (50 ml) and with water (200 ml) to give a white solid (27.20 g, 65%), mp: 115°C.

¹H-NMR (DMSO d₆) : 3.65 (s, 3H), 4.30 (s, 2H), 4.57 (s, 2H), 6.77 (d, 2H), 6.94 (d, 2H), 7.48 (d, 2H), 7.76 (d, 2H).

### PREPARATION OF IMIDAZOLES (9)

### EXAMPLE 2

### 4-[N-(1H-imidazol-1-yl)amino]benzonitrile

### a) 4-[N-(2,3-dihydro-1H-imidazol-1-yl-2-thione)amino]benzonitrile

To a suspension of 4-cyanophenylhydrazine hydrochloride (6.00 g, 35.40 mmol) in ethanol (60 ml) was added dropwise 2,2-dimethoxyethylisothiocyanate (6.25 g, 42.4 mmol) and the reaction mixture was heated to reflux for 2 h. After cooling the solvent was evaporated under vacuum, the resulting oil was diluted with acetic acid/ water (9/1, 32 ml) and the suspension was heated to reflux for 1.5 h and at room temperature overnight. The resulting residue was poured into water (300 ml) and a brown precipitate was collected. After trituration from ethanol, the brown solid afforded a white solid (4.60 g, 58 %).

¹H-NMR (DMSO d₆) : 6.54 (d, 2H), 7.00 (t, 1H), 7.23 (t, 1H), 7.62 (d, 2H), 9.83 (s, 1H), 12.40 (s, 1H).

### b) 4-[N-(1H-imidazol-1-yl)amino]benzonitrile

35 % hydrogen peroxide (4.90 ml, 55.5 mmol) was added dropwise to an ice-cooled suspension of 4-[N-(2,3-dihydro-1H-imidazol-1-yl-2-thione)amino]benzonitrile (4.00 g, 18.50 mmol) in acetic acid (20 ml). When TLC showed complete reaction, the reaction mixture was diluted with water, adjusted to pH 11 with sodium hydroxide, treated with sodium hydrogen sulfite and extracted with ethyl acetate. The organic layer was dried over sodium sulfate and concentrated under vacuum. Flash chromatography on silica gel (toluene / dioxane : 6/4) yielded a pure oil and crystallization from ethanol afforded white crystals (4.40 g, 58 %), mp: 162°C.

¹H-NMR (DMSOd₆) : 6.50 (d, 2H), 7.08 (s, 1H), 7.30 (s, 1H), 7.66 (d, 2H), 7.83 (s, 1H).

### PREPARATION OF IMIDAZOLES OF FORMULA (I)

Using the same procedure as described in example 2, but replacing the 4-cyanophenylhydrazine, hydrochloride by:
- N¹-(4-cyanophenylmethyl)-N¹-(4-methoxyphenyl)hydrazine,
the following compound was obtained:

### EXAMPLE 3

4-[N-(1H-imidazol-1-yl)-N-(4-methoxyphenyl)amino]methylbenzonitrile

¹H-NMR (DMSO d₆) : 3.70 (s, 3H), 4.90 (s, 2H), 6.60-7.00 (m, 5H), 7.40 (s, 1H), 7.55 (d, 2H), 7.70 (s, 1H), 7.78 (d, 2H).

Crystallization from hydrochloric ethanol yielded white crystals (5.70 g, 66 %). mp: 207°C

¹H-NMR (DMSO d₆) : 3.70 (s, 3H), 4.97 (s, 2H), 6.93 (d, 2H), 7.13 (d, 2H), 7.45 (d, 2H), 7.70 (s, 1H), 7.84 (d, 2H), 8.04 (s, 1H), 8.18 (s, 1H), 9.55 (s, 1H).

### EXAMPLE 4

4-[N-(4-hydroxyphenyl)-N-(1H-imidazol-1-yl)amino]methylbenzonitrile

A solution of boron tribromide (60 ml, 60.00 mmol) in 20 ml of dichloromethane is added to a cold (0-5°C) solution of 4-[N-(1H-imidazol-1-yl)-N-(4-methoxyphenyl)amino]methylbenzonitrile (4.60 g, 15.11 mmol). After 1 h at room temperature the mixture was hydrolysed with saturated aqueous NaHCO₃, filtered, washed with water (50 ml) and with dichloromethane (20 ml) to give a brown solid (4.00 g). Crystallization from acetone yielded a brown solid (3.00 g, 68%) mp: 150°C.

¹H-NMR (DMSO d₆) : 4.84 (s, 2H), 6.70 (s, 4H), 6.90 (s, 1H), 7.45-7.62 (m, 3H), 7.62-7.90 (m, 3H), 9.25 (s, 1H).

### EXAMPLE 5

4-[N-(4-hydroxyphenylmethyl)-N-(1H-imidazol-1-yl)amino]benzonitrile

4-hydroxybenzylbromide (15.6 g, 84.3 mmol, prepared following Wissner A. et al., J.Med.Chem. 1992, 35, 1650) was added to a mixture of 4-[N-(1H-imidazol-1-yl)amino]benzonitrile (10.00 g, 54.30 mmol) and K₂CO₃ (8.20 g, 59.70 mmol) in dry THF (150 ml) at room temperature. The mixture was then stirred at room temperature for 2 h and after poured into water and extracted with ethyl acetate, dried over Na₂SO₄, filtered, and concentrated under vacuum to give the crude product (16.00 g as solid). Crystallization from ethyl acetate with ethanol yielded the expected product (6.50 g, 41%, mp: 180°C).

¹H-NMR (DMSO d₆) : 4.80 (s, 2H), 6.65 (d, 2H), 6.91 (s, 1H), 7.04 (d, 1H), 7.20 (s, 1H), 7.56 (s, 1H), 7.63 (d,2H).

Using the same procedure but replacing the 4-hydroxybenzylbromide by:
- 3-chloro-4-hydroxybenzylbromide
- 3-bromo-4-hydroxybenzylbromide
- 4-hydroxy-3-methoxybenzylbromide
- 2,3,5,6-tetrafluoro-4-hydroxybenzylchloride (prepared following Angyal S.J. et al., J.Chem. Soc. 1950, 2141)
- 3-formyl-4-hydroxybenzylchloride (prepared following Angyal S.J. et al., J.Chem. Soc. 1950, 2141)
the following compounds were respectively obtained:

### EXAMPLE 6

4-[N-(3-chloro-4-hydroxyphenylmethyl)-N-(1H-imidazol-1-yl)amino]benzonitrile

mp 195°C

¹H-NMR (DMSOd₆) : 4.88 (s, 2H), 6.67 (d, 2H), 6.88 (d, 2H), 6.98 (s, 1H), 7.05 (dd, 1H), 7.24 (d, 1H), 7.33 (s, 1H), 7.70 (s,1H), 7.72 (d,2H), 10.28 (s, 1H).

### EXAMPLE 7

4-[N-(3-bromo-4-hydroxyphenylmethyl)-N-(1H-imidazol-1-yl)amino]benzonitrile

mp 198°C

¹H-NMR (DMSOd₆) : 4.90 (s, 2H), 6.65 (d, 2H), 6.85 (d, 1H), 6.99 (s, 1H), 7.07 (d, 1H), 7.30 (s, 1H), 7.40 (s, 1H), 7.65 (s,1H), 7.67 (d,2H), 10.40 (s, 1H).

### EXAMPLE 8

4-[N-(4-hydroxy-3-methoxyphenylmethyl)-N-(1H-imidazol-1-yl)amino]benzonitrile

mp 215°C

¹H-NMR (DMSOd₆) : 3.70 (s, 3H), 4.89 (s, 2H), 6.68 (s, 2H), 6.70 (d, 2H), 6.80 (s, 1H), 6.99 (s, 1H), 7.30 (s, 1H), 7.63 (s, 1H), 7.72 (d,2H), 9.20 (s, 1H).

### EXAMPLE 9

4-[N-(2,3,5,6-tetrafluoro-4-hydroxyphenylmethyl)-N-(1H-imidazo)-1-yl)amino]benzonitrile

mp 243°C

¹H-NMR (DMSOd₆): 5.09 (s, 2H), 6.72 (d, 2H), 7.00 (s, 1H), 7.32 (s, 1H), 7.69 (d, 2H), 7.77 (s, 1H), 11.80 (s, 1H).

### EXAMPLE 10

4-[N-(3-formyl-4-hydroxyphenylmethyl)-N-(1H-imidazol-1-yl)amino]benzonitrile

mp 160°C

¹H-NMR (DMSOd₆) : 4.95 (s, 2H), 6.70 (d, 2H), 6.90 (s, 1H), 6.96 (d, 1H), 7.35 (s, 1H), 7.44 (dd, 1H), 7.58 (d, 1H), 7.67 (s, 1H), 7.71 (d, 2H), 10.20 (s, 1H), 10.75 (s, 1H).

### EXAMPLE 11

4-[N-(1H-imidazol-1-yl)-N-(4-nitrophenyl)amino]benzonitrile

4-[N-(1H-imidazol-1-yl)amino]benzonitrile (10.00 g, 54.30 mmol) was added portionwise to a suspension of potassium tert-butoxide (6.69 g, 59.73 mM) in DMSO (100 ml) at ( 10-15°C) with stirring. The mixture was stirred for 30 mn at room temperature, and then 4-nitro-fluorobenzene (7.60 g, 54.00 mM) in DMSO (15 ml) was added dropwise while keeping the temperature below 30°C. After 2h, the mixture was poured into water (800 ml) and the resulting precipitate was collected by filtration and purified by crystallization from ethanol (1.00 g, 48%, mp: 188°C).

¹H-NMR (DMSOd₆) : 7.00 (d, 2H), 7.17 (s, 1H), 7.26 (d, 2H), 7.65 (s, 1H), 7.90 (d, 2H), 8.20 (s,1H), 7.22 (d,2H).

### EXAMPLE 12

4-[N-(4-aminophenyl)-N-(1H-imidazol-1-yl)amino]benzonitrile

Hydrazine (1.52 ml, 49.00 mmol) was added portionwise to a suspension of 4-[N-(1H-imidazol-1-yl)-N-(4-nitrophenyl)amino]benzonitrile (3.00 g, 9.80 mM) and ruthenium, 5 wt. % on carbon (0.30 g, 0.15 mM) in ethanol (35 ml) at reflux with stirring. When TLC showed complete reaction, the mixture was cooled and the catalyst was filtered. The solvent was concentrated under vacuum. The residue was poured into water and extracted with dichloromethane, dried over Na₂SO₄, filtered, and concentrated under vacuum to give the crude product (2.50g as solid). Crystallization from ethyl acetate with ethanol yielded the expected product (1.30 g, 50%, mp: 147°C).

¹H-NMR (DMSOd₆) : 5.50 (s, 2H), 6.30 (d, 2H), 6.69 (d, 2H), 7.09 (s, 1H), 7.29 (d, 2H), 7.63 (s, 1H), 7.65 (d, 2H), 8.14 (s,1H).

### GENERAL PROCEDURE OF SULFAMOYLATION

### EXAMPLE 13

Sulfamic acid 4-[N-(4-cyanophenylmethyl)-N-(1H-imidazol-1yl)amino]phenyl ester Sulfamoyl chloride (2.39 g, 20.69 mmol) was added to a solution of 4-[N-(4-hydroxyphenyl)-N-(1H-imidazol-1-yl)amino]methylbenzonitrile (1.00 g, 3.45 mmol) in dry DMAc (36 ml) with ice cooling. The mixture was then stirred at room temperature for 6 h. After addition of TEA (3.40 ml, 24.73 ml), the mixture was poured into cold brine and extracted with ethyl acetate, dried over Na₂SO₄, filtered, and concentrated under vacuum to give the crude product (0.70 g as solid). Crystallization from ethyl acetate yielded the expected product (0.40 g, 31%, mp: 60°C).

¹H-NMR (DMSO d₆) : 5.00 (s, 2H), 6.70 (d, 2H), 6.92 (s, 1H), 7.19 (d, 2H), 7.40 (s, 1H), 7.55 (d, 2H), 7.74 (s, 1H), 7.77 (d, 2H), 7.91 (s, 2H).

Using the same procedure but replacing the 4-[N-(4-hydroxyphenyl)-N-(1H-imidazol-1-yl)amino]methylbenzonitrile by:
- 4-[N-(4-hydroxyphenylmethyl)-N-(1H-imidazol-1-yl)amino]benzonitrile
- 4-[N-(3-chloro-4-hydroxyphenylmethyl)-N-(1H-imidazol-1-yl)amino]benzonitrile
- 4-[N-(3-bromo-4-hydroxyphenylmethyl)-N-(1H-imidazol-1-yl)amino]benzonitrile
- 4-[N-(3-methoxy-4-hydroxyphenylmethyl)-N-(1H-imidazol-1-yl)amino] benzonitrile
- 4-[N-(2,3,5,6-tetrafluoro-4-hydroxypheny)methy))-N-(1H-imidazo)-1-yl)amino] benzonitrile
- 4-[N-(3-formyl-4-hydroxyphenylmethyl)-N-(1H-imidazol-1-yl)amino] benzonitrile
- 4-[N-(4-aminophenyl)-N-(1H-imidazol-1-yl)amino]benzonitrile
- 4-[N-(1H-imidazol-1-yl)amino]benzonitrile
the following compounds were respectively obtained:

### EXAMPLE 14

Sulfamic acid-4-{[N-(4-cyanophenyl)-N-(1H-imidazol-1yl)amino]methyl}phenyl ester

mp 172°C

¹H-NMR (DMSOd₆) : 5.00 (s, 2H), 6.65 (d, 2H), 7.00 (s, 1H), 7.22 (d, 2H), 7.40 (s, 1H), 7.42 (d, 2H), 7.70 (s, 1H), 7.75 ( d, 2H), 8.00 (s, 2H).

### EXAMPLE 15

Sulfamic acid 2-chloro-4-{[N-(4-cyanophenyl)-N-(1H-imidazol-1yl)amino]methyl}phenyl ester

¹H-NMR (DMSOd₆) : 5.05 (s, 2H), 6.63 (d, 2H), 7.00 (s, 1H), 7.35-7.45 (m, 3H), 7.54 (s, 1H), 7.70 (d, 2H), 7.80 (s, 1H), 8.29 (s, 2H).

### EXAMPLE 16

Sulfamic acid 2-bromo-4-{[N-(4-cyanophenyl)-N-(1H-imidazol-1yl)amino]methyl}phenyl ester

¹H-NMR (DMSOd₆) : 5.07 (s, 2H), 6.66 (d, 2H), 7.03 (s, 1H), 7.44 (s, 3H), 7.70 (d, 2H), 7.80 (d, 1H), 8.28 (s, 2H).

### EXAMPLE 17

Sulfamic acid 2-methoxy-4-{[N-(4-cyanophenyl)-N-(1H-imidazol-1yl)amino]methyl}phenyl ester

mp 211°C

¹H-NMR (DMSOd₆) : 3.77 (s, 3H), 5.02 (s, 2H), 6.75 (d, 2H), 6.92 (d, 1H), 7.05 (s, 2H), 7.25 (d, 1H), 7.45 (s, 1H), 7.71 (d, 2H), 7.80 (s, 1H), 7.93 (s, 2H).

### EXAMPLE 18

Sulfamic acid 2,3,5,6-tetrafluoro-4-{[N-(4-cyanophenyl)-N-(1H-imidazol-1yl)amino]methyl}phenyl ester

¹H-NMR (DMSOd₆) : 5.37 (s, 2H), 5.83 (s, 2H), 6.86 (d, 2H), 7.08 (s, 1H), 7.41 (s, 1H), 7.79 (s, 1H), 7.81 (d, 2H).

### EXAMPLE 19

4-[N-[(2,2-dioxido-1,2,3-benzoxathiazin-6-yl)methyl]-N-(1H-imidazol-1-yl)amino] benzonitrile

mp 180°C

¹H-NMR (DMSOd₆) : 5.17 (s, 2H), 6.67 (d, 2H), 7.02 (s, 1H), 7.46 (s, 1H), 7.50 (d, 2H), 7.74 (d, 2H), 7.82 (dd, 1H), 7.87 (s, 1H), 8.02 (d, 1H), 9.19 (s, 1H).

### EXAMPLE 20

N-{4-[N-(4-cyanophenyl)-N-(1H-imidazol-1-yl)amino]phenyl}sulfamide

mp 121°C

¹H-NMR (DMSOd₆) : 6.38 (d, 2H), 7.09 (s, 1H), 7.20 (s, 1H), 7.22 (d, 2H), 7.51 (d, 2H), 7.63 (s, 1H), 7.69 (d, 2H), 8.19 (s, 1H)

### EXAMPLE 21

4-[N-[(2,2-dioxido-3,4-dihydro-1,2,3-benzoxathiazin-6-yl)methyl]-N-(1H-imidazol-1-yl)amino]benzonitrile

To a suspension of 4-[N-[(2,2-dioxido-1,2,3-benzoxathiazin-6-yl)methyl]-N-(1H-imidazol-1-yl)amino]benzonitrile (0.40 g, 1.05 mmol) in methanol (8 ml) was added portionwise NaBH₄ (0.08 g, 2.11 mmol) and the reaction mixture was stirred at room temperature for 3 h. After addition of a saturated solution of NH₄Cl (3 ml) and water (40 ml), the precipitate obtained was filtered, washed with water and dried to give a white solid. Crystallization from ethyl acetate with ethanol yielded the expected product (0.40 g, 82%, mp: 190°C).

¹H-NMR (DMSOd₆) : 4.55 (d, 2H), 5.04 (s, 2H), 6.64 (d, 2H), 7.02 (s, 1H), 7.03 (d, 1H), 7.27 (d, 1H), 7.30 (s, 1H), 7.41 (s, 1H), 7.70 (d, 2H), 7.85 (s, 1H), 8.56 (t, 1H).

### BIOLOGICAL TEST RESULTS

### INHIBITION OF STEROID SULFATASE, AROMATASE AND CARBONIC ANHYDRASE II IN VITRO

Estrone sulfate (E₁S) is a major circulating plasma estrogen that is converted by the steroid sulfatase enzyme into estrone (E₁), which in turn can be transformed into estradiol (E₂) by enzymatic reduction.

The reduction of E₂ synthesis by aromatase inhibitors has been clinically proved to be the best way to halt the progress of hormone-dependent breast tumors. In the following experiments, we have shown that compounds with both aromatase and steroid sulfatase activities are a suitable approach to inhibit tumor growth.

Human carbonic anhydrases catalyse the conversion between carbon dioxide (CO₂) and the bicarbonate ion (HCO₃⁻), and are involved in physiological and pathological processes. They includes hormone-dependent and non-hormone-dependent cancerogenesis, metastasis process and hypoxic tumors which express these enzymes that are less responsive to classical chemo/radio-therapy. In particular, EMATE was found to have a human carbonic anhydrases inhibitory potency similar to that of acetazolamide, a well-known sulfonamide human carbonic anhydrases inhibitor. The aim of these experiments was to evaluate the in vitro inhibitory potential of aromatase and/or steroid sulfatase inhibitors on human carbonic anhydrase II activity (as an exemple of human carbonic anhydrase) in comparison with 6,6,7 COUMATE and acetazolamide.

### Aromatase activity, Methods & Materials

The JEG-3 cell line over-expresses human aromatase. 24 hours before measurement, cells are distributed for seeding in 96-well microplates, 24 hours later, microplates are rinsed and fresh medium containing the radioactive aromatase substrate (1β⁻³H-androstenedione, 10 nM) is added together with test compounds dissolved with 1 % dimethylsulfoxyde in a test concentration range between 10⁻¹¹ and 10⁻⁴ M in a total volume of 150 µl. Two hours after the beginning of the incubation, 100 µl of the supematants are transferred to homologous new 96-well microplates. A solution of dextran-coated charcoal (1 %) is added in each well (100 µl/well); after standing on ice for 10 minutes, microplates are centrifuged (1500 g; 4°C). All steroids, including the radioactive substrate and the newly biosynthesized estrogens, are adsorbed on charcoal; only ³H-water specifically formed during aromatisation of 1β⁻³H-androstenedione, involving a specific oxidative step removing the 1β⁻³H, remains in the supernatant at this stage. Supernatants received a scintillation cocktail for β-radioactivity measurement. In parallel, the aromatase reaction is stopped in the microplates by destruction and solubilization of the cells in a 10 mM ethylenediamine tetraacetate solution at pH 12.3. Then, DNA is measured by a standard fluorimetric method using the Hoechst 33258 fluorochrome and a Victor² (Wallac, EG & G) microplate fluorimeter. Finally, aromatase activity is expressed in fmoles/µg DNA in 2 hours and aromatase inhibition as a percentage of that observed in a control incubation without inhibitors. A non-linear fit analysis of % of inhibition *vs*. concentration allowed the determination of the 50 % inhibitory concentration (IC₅₀) : the lowest IC₅₀ correspond to the most potent inhibitors **(Table 1).**

### Steroid sulfatase activity, Methods & Materials

The JEG-3 cell line is very rich in human estrone sulfatase. Assays were carried out with cells in logarithmic growth phase on 96-well microplates. 24h before studies, cells were seeded in decomplemented fetal calf serum (dFCS) supplemented medium. Then, the seeding medium was removed and the cells were rinsed with PBS to eliminate any trace of dFCS. Then ³H-E₁S was added, followed by test compounds at concentrations ranging from 10⁻¹² M to 10⁻⁵ M. After 4 h of treatment, the medium was transferred into 96-deep-well microplates and centrifuged at 200 x g for 10 min to pellet cells before toluene extraction. A fraction of medium was used for toluene extraction in order to separate the conjugated substrate from non-conjugated products. The radioactivity in the toluene phase was measured by liquid scintillation counting. Finally, estrone sulfatase activity was expressed in pmoles of ³H-E₁ + ³H-E₂ formed per 4 hours and per µg DNA and estrone sulfatase inhibition as a percentage of control activity without inhibitor. A non linear fit analysis (GraphPad Prism Software) of % inhibition *vs*. inhibitor concentrations allowed the determination of the 50 % inhibitory concentration (IC₅₀): the lowest IC₅₀ corresponds to the most potent inhibitors (Table 1).

### Alkaline phosphatase activity, Methods & Materials

To evaluate the estrogenic potency of test compounds, the well-established Ishikawa cell model was used. Cells were routinely grown in a medium containing 10% dFCS. Seeding was performed in Nunc culture plastic flasks and cells were maintained in a humidified atmosphere of 5% CO₂ and 95% air at 37±0.1 C. The effect of the test compounds on alkaline phosphatase induction were measured in estrogen-free medium. Briefly, cells were seeded into 96-well microplate 48 h before studies. At the end of a 4-day treatment alkaline phosphatase activity was measured with the *p*-nitrophenyl-phosphate assay, and the activity was expressed as n-fold increase with respect to control levels, then as a percentage of the activity recorded in the presence of 10⁻⁸ M E₂, which was taken to be equal to 100% **(Table 1).**

### Human carbonic anhydrase II activity, Methods & Materials

This assay was performed as described in the literature (Armstrong J. et al. Purification and properties of human erythrocyte carbonic anhydrases, J Biol Chem, 1966, 241: 5137-5149). Briefly, in this assay, human carbonic anhydrase II catalyses the conversion of *p*-nitrophenyl acetate into *p*-nitrophenol. The potential inhibitory effect of test compounds was evaluated by colorimetric determination of *p*-nitrophenolproduced during the enzymatic reaction. The optical density levels obtained without inhibitor will be referred to as "total activity". The levels obtained without inhibitor and without the enzyme will be referred to as "blank" in order to assess any interference with the substrate during the assay **(Table 2)**.

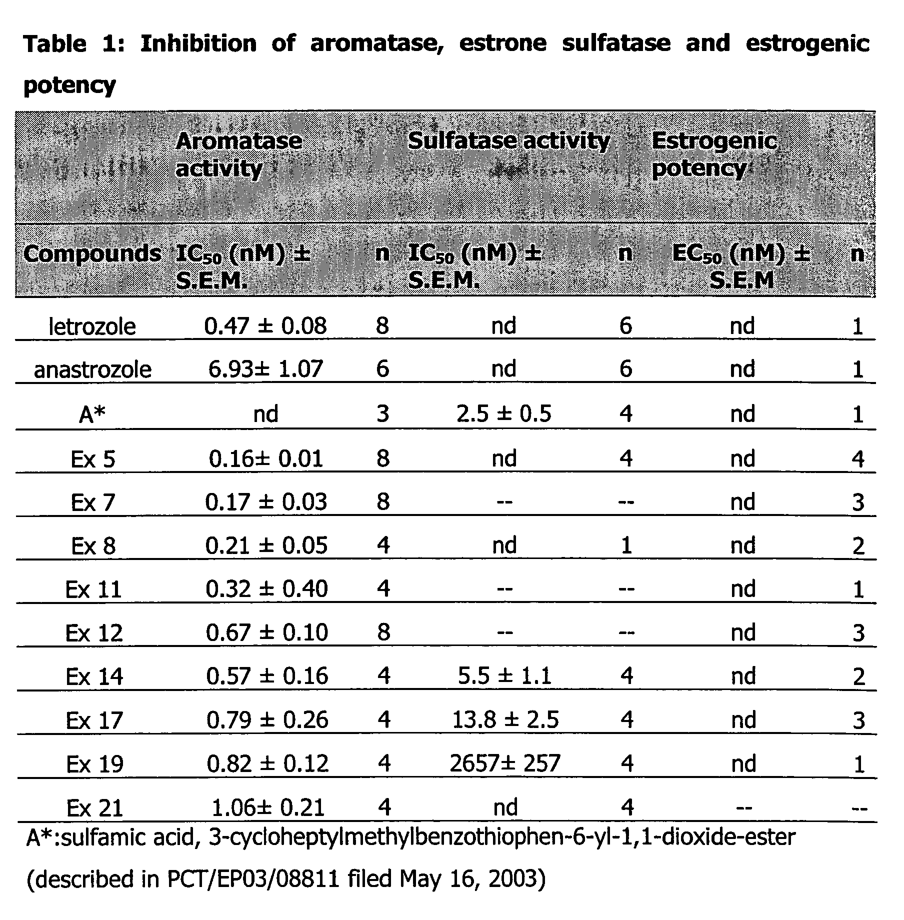

nd: not detected

Among the tested compounds, Ex 14 and Ex 17 showed a strong inhibition (IC₅₀ about 10 nM) of human estrone sulfatase activity. In addition, the same compounds were shown to be strong inhibitors of aromatase activity. Despite this dual activity, Ex 14 and Ex 17 were not estrogenic *in vitro*.

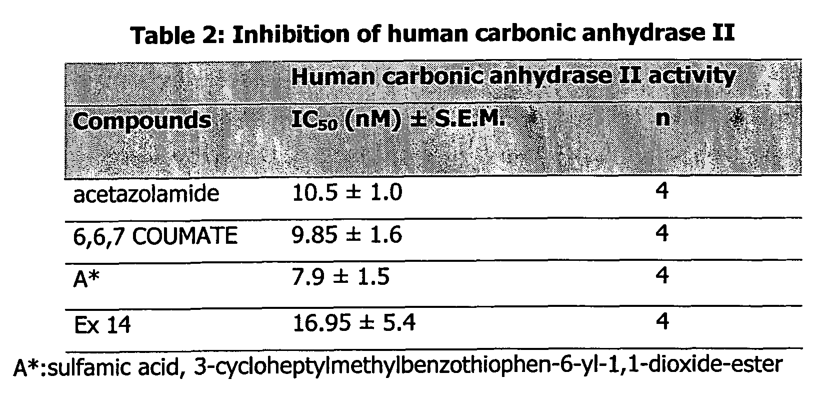

Ex 14 inhibited human carbonic anhydrase II *in vitro.*

### ANTI UTEROTROPHIC/ANTI STEROID SULFATASE ACTIVITY IN VIVO

Wistar female rats were ovariectomized and left to rest for 4 weeks. Prior to treatment, the absence of cyclicity was checked by vaginal smears. Animals were supplemented with estrone sulfate (E₁S) at 50 µg/kg/day s.c., alone or combined with oral administration of potential sulfatase inhibitors, at 1 mg/kg/day for 4 days. The uteri were removed, freed of adjacent tissue and wet weighed (**Table 3**).

Estrone sulfatase activity was measured according to the method described by Purohit et al., with slight modifications. Briefly, uteri were thawed, weighed and homogenized. Aliquots of the supernatant were treated with dextran-coated charcoal and assayed for sulfatase. E₁S activity was assessed after 30 min of incubation with 5 nM of ³H-E₁S and 20 µM of unlabelled E₁S as substrate. Estrone sulfatase activity was expressed as pmol /h/mg protein and reported as percentage of inhibition *vs* E₁S; for uteri weights, results are expressed as % of inhibition of the E₁S induced stimulation **(Table 3).**

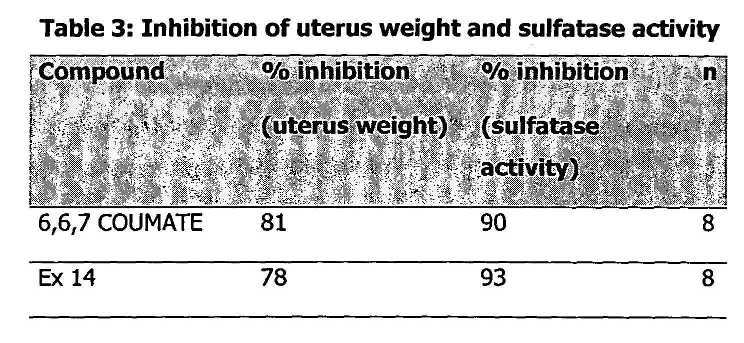

Ex 14 was chosen as a potential inhibitor of estrone sulfatase activity because of its lack of estrogenicity, significant inhibition of E₁S stimulated uterus weight and complete inhibition of tissue estrone sulfatase activity. These *in vivo* results were in agreement with *in vitro* results obtained in JEG-3 cells.

### INHIBITION OF TUMOR GROWTH

### MCF-7 xenograft into nude mice

MCF-7 cells, derived from human breast adenocarcinoma, were injected subcutaneously in ovariectomized athymic nude mice supplemented with a daily administration of subcutaneous estrone sulfate. Xenograft volumes were determined weekly. When tumor volumes reached significant increase, Ex 14 was orally administered at 1mg/kg/day for 8 weeks. Xenografts were measured, removed, weighed, and deep frozen until the determination of sulfatase activity, according the above-mentioned methods **(Table 4)**.

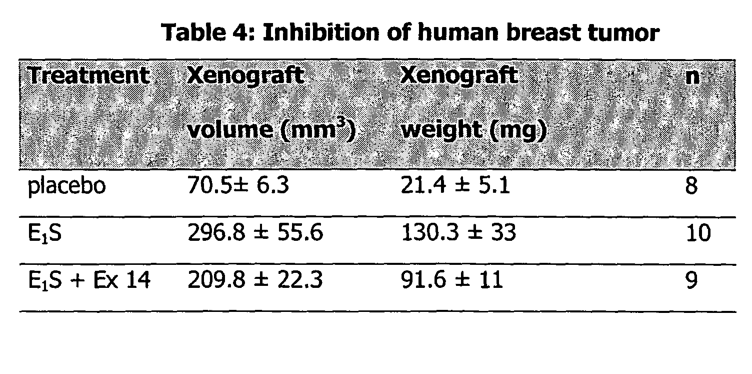

30% inhibition of breast tumor were obtained with Ex 14 after 8 weeks of oral administration at 1mg/kg/day.

### JEG-3 xenograft into nude mice

The JEG-3 cell line over-expresses both human aromatase and estrone sulfatase enzymes. It was injected subcutaneously into ovariectomized athymic nude mice supplemented with a daily administration of subcutaneous estrone sulphate with or without Ex 14 (administered at 5mg/kg/day). In that particular case, and according to their semi-liquid status (choriocarcinoma origin), tumor measures were irrevelant. Nevertheless, an indirect effect of estradiol which was mainly synthetised by the tumour tissue, was obtained on uteri weights. On the other hand, because of the aromatase and sulfatase overexpression in such tumours, enzyme levels were measurable. Enzyme activities were determined according to the above-mentioned methods **(Table 5)**.

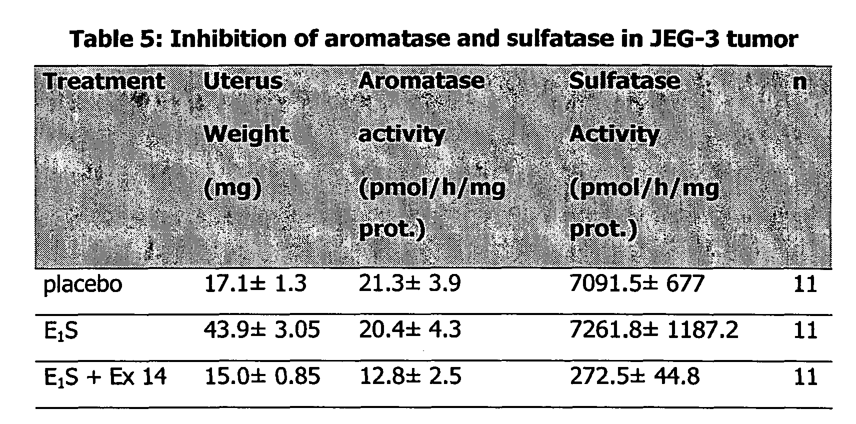

37.3% inhibition of aromatase activity was obtained with Ex 14 after 12 days of oral administration at 5mg/kg/day, the same compound led to a sulfatase activity decrease of 96.2%.

### JEG-3 xenograft within nude rat supplemented with estrone sulphate and Δ4-androstenedione

15 days before JEG-3 cell injection into rats, blood samples were taken to measure basal levels of estradiol plasma. Then, JEG-3 cells were injected subcutaneously in ovariectomized athymic (Rnu/Rnu) nude rat. Animals were supplemented with a daily administration of subcutaneous estrone sulphate and Δ4-androstenedione with or without Ex 14 (administered at 1mg/kg/day). After a 21-day period, blood sampling were performed one day after cancerous xenograft and at the end of the experiment. In this experiment estrone sulphate and Δ4-androstenedione are the precursors of estradiol. The effect of estradiol was reflected on the uterus weight after sacrifice. Plasma hormone levels were assayed at the end of the experiments according to the supplier's standard method (DSL,Webster,TX,USA) **(Table 6).**

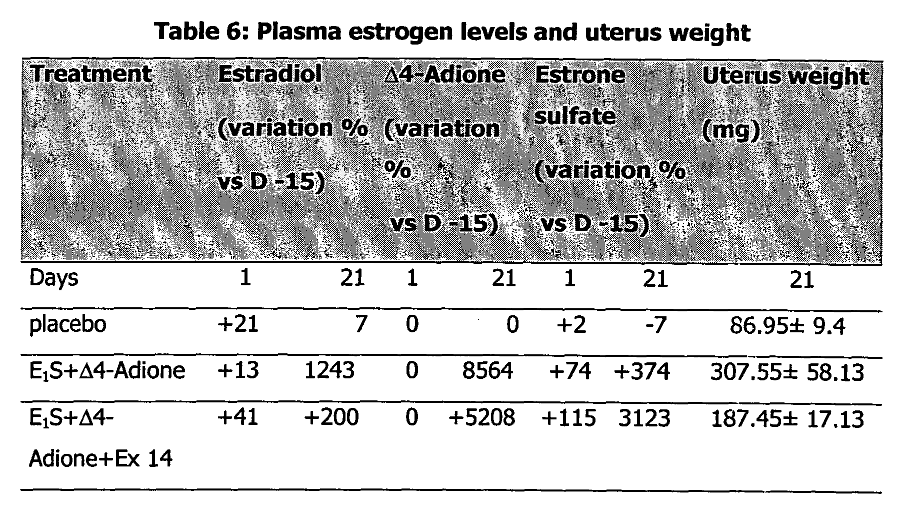

The results are expressed as variation percentage of initial value mesured at day -15 (i.e.: 15 days before cancerous cell injection). Ex 14 led to a decrease in estradiol plasma level, as expected, in respect to its effect on tumor aromatase and sulfatase inhibition. Finally, Ex14 shown an anti-uterotrophic potency, when animals were supplemented with estrone sulfate and Δ4-androstenedione.

## Claims

1. An imidazole derivative of formula (I) : 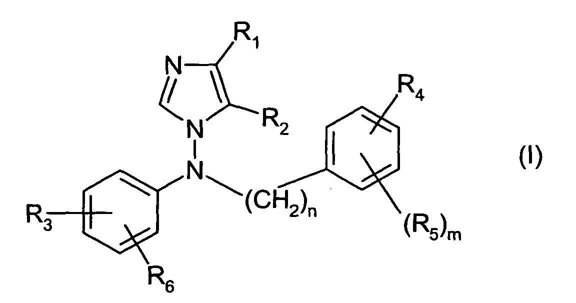 and acid addition salts and stereoisomeric forms thereof, wherein :
• R₁ and R₂ are each independently hydrogen, a (C₁-C₆)alkyl or a (C₃-C₈)cycloalkyl ; or R₁ and R₂ together form a saturated or unsaturated 5-, 6- or 7- membered carbocyclic ring;
• n is 0, 1 or 2 ;
• one of R₃ and R₄ is a hydroxy, nitro, -NR₇R₈, -OSO₂NR₇R₈ or -NR₇SO₂NR₇R₈ group and the other is hydrogen or a hydroxy, cyano, halogen, nitro, (C₁-C₆)alkyl, (C₁-C₆)alkoxy, trifluoromethyl, (C₁-C₆)alkylthio, (C₁-C₆)alkylsulfonyl, acyl, (C₁-C₆)alkoxycarbonyl, carboxamido, -NR₇R₈, -OSO₂NR₇R₈ or - NR₇SO₂NR₇R₈ group;
• R₅ and R₆ are each independently hydroxy, cyano, halogen, nitro, (C₁-C₆)alkyl, (C₁-C₆)alkoxy, trifluoromethyl, (C₁-C₆)alkylthio, (C₁-C₆)alkylsulfonyl, acyl, (C₁-C₆)alkoxycarbonyl, carboxamido, -NR₇R₈, -OSO₂NR₇R₈ or - NR₇SO₂NR₇R₈ group ;
• R₇ and R₈ are each independently hydrogen, a (C₁-C₆)alkyl or a (C₃-C₈)cycloalkyl;
• m is 1, 2, 3 or 4;
• R₄ and R₅ together with the phenyl ring bearing them can also form a benzoxathiazine dioxide or a dihydrobenzoxathiazine dioxide;
• R₃ and R₆ together with the phenyl ring bearing them can also form a benzofurane or a N-methylbenzotriazole.

2. A derivative according to claim 1, and acid addition salts and stereoisomeric forms thereof, wherein:
• n is 0 or 1;
• R₁ and R₂ are each independently hydrogen or (C₁-C₆)alkyl;
• R₅ and R₆ are each independently hydrogen, halogen, (C₁-C₆)alkoxy, acyl, - NR₇R₈, -OSO₂NR₇R₈ or -NR₇SO₂NR₇R₈.

3. A derivative according to claim 1 or 2, and acid addition salts and stereoisomeric forms thereof, wherein:
• n is 0 or 1;
• R₁, R₂ and R₆ are each hydrogen;
• R₅ is hydrogen, halogen, (C₁-C₆)alkoxy or -OSO₂NR₇R₈.

4. A derivative according to one of claims 1 to 3, and acid addition salts and stereoisomeric forms thereof, wherein one of R₃ and R₄ is hydroxy, cyano or - OSO₂NR₇R₈ and the other is hydroxy, nitro, -NR₇R₈, -OSO₂NR₇R₈ or - NR₇SO₂NR₇R₈.

5. A derivative according to claim 4, and acid addition salts and stereoisomeric forms thereof, wherein one of R₃ and R₄ is cyano or -OSO₂NR₇R₈ and the other is hydroxy or -OSO₂NR₇R₈.

6. A derivative according to one of claims 1 to 5, and acid addition salts and stereoisomeric forms thereof, wherein R₇ and R₈ are hydrogen.

7. A derivative according to one of claims 1 to 6, and acid addition salts and stereoisomeric forms thereof, wherein n is 1.

8. A derivative according to one of claims 1 to 7, and acid addition salts and stereoisomeric forms thereof, wherein m is 1.

9. A pharmaceutical composition comprising a derivative according to one of claims 1 to 8, or a pharmaceutically acceptable acid addition salt thereof, and a pharmaceutical acceptable carrier.

10. A pharmaceutical composition according to claim 9, comprising from 0.1 to 400 mg of said derivative.

11. Use of a derivative according to one of claims 1 to 8, or a pharmaceutically acceptable acid addition salt thereof, as a pharmaceutical.

12. Use of a derivative according to one of claims 1 to 8 or a pharmaceutically acceptable acid addition salt thereof in the manufacture of a medicament for the treatment or prevention of hormone- or non hormone-dependent tumors, wherein said derivative is optionally combined with a sexual endocrine therapeutic agent.

13. Use of a derivative according to one of claims 1 to 8 or a pharmaceutically acceptable acid addition salt thereof in the manufacture of a medicament for the control or management of reproductive functions, wherein said derivative is optionally combined with a LH-RH agonist or antagonist, an estroprogestative contraceptive, a progestin, an anti-progestin or a prostaglandin.

14. Use of a derivative according to one of claims 1 to 8 or a pharmaceutically acceptable acid addition salt thereof in the manufacture of a medicament for the treatment or prevention of benign or malignant diseases of the breast, the uterus or the ovary, wherein said derivative is optionally combined with an antiandrogen, an anti-estrogen, a progestin or a LH-RH agonist or antagonist.

15. Use of a derivative according to one of claims 1 to 8 or a pharmaceutically acceptable acid addition salt thereof in the manufacture of a medicament for the treatment or prevention of androgen-dependent diseases or benign or malignant diseases of the prostate or the testis, wherein said derivative is optionally combined with an antiandrogen, a progestin, a lyase inhibitor or a LH-RH agonist or antagonist.

16. Use of a derivative according to one of claims 1 to 8 or a pharmaceutically acceptable acid addition salt thereof in the manufacture of a medicament for the treatment or prevention of cognitive function disorders, especially senile dementia, in particular Alzheimer's disease.

17. Use of a derivative according to one of claims 1 to 8 or a pharmaceutically acceptable acid addition salt thereof in the manufacture of a medicament for the treatment or prevention of immunodisorders.

18. Use of a derivative according to one of claims 1 to 8 or a pharmaceutically acceptable acid addition salt thereof in the manufacture of a medicament for the treatment or prevention of pathologies in which inhibition of aromatase and/or steroid sulfatase and/or carbonic anhydrase is required.
